# EUROPEAN PATENT SPECIFICATION

(11) **EP 1 935 385 B1**
(45) Date of publication and mention of the grant of the patent: **04.05.2011**
(21) Application number: 07122888.6
(22) Date of filing: 11.12.2007
(51) Int. Cl.: A61F 9/01, A61F 9/008

(54) **Performance and accuracy assessment system for refractive laser systems**
Leistungs- und Genauigkeitsprüfsystem für refraktive Lasersysteme
Système d'évaluation de la performance et de l'exactitude pour systèmes lasers de réfraction

(30) Priority: 19.12.2006 US 612721
(43) Date of publication of application: 25.06.2008
(73) Proprietor: Alcon RefractiveHorizons, Inc., Fort Worth, Texas 76134 (US)
(72) Inventor: Leblanc, Richard Alan, Clermont, FL 34715-7706 (US); Sensiper, Martin, Orlando, FL 32806 (US); Nguyen, Phuoc Khanh, Winter Springs, FL 32708 (US)
(74) Representative: Hanna, Peter William Derek

(56) References cited:
- EP-A- 1 273 279
- DE-A1- 10 335 303
- US-A- 5 928 221
- US-A1- 2002 120 198
- US-A1- 2005 021 011

## Description

### FIELD OF INVENTION

The present invention generally relates to ophthalmic laser surgery and, in particular, to systems for assessing system component performance, and, most particularly, to systems for assessing the performance and accuracy of a refractive laser system.

### BACKGROUND

Ophthalmic surgery for the correction of vision is known to be performed with lasers, for example, excimer lasers, that are used to ablate the cornea in a predetermined pattern. The laser is applied in bursts of energy, referred to as shots, each of which has physical and temporal characteristics that must be known precisely in order that the predetermined pattern be commensurate with the pattern ablated by the laser. Further, the optical system that directs the shots to the cornea also has physical characteristics that must be precisely calibrated in order that the shots be placed correctly.

Currently known methods for assessing laser system performance are typically not performed during surgery, and instead laser statistics are monitored during calibration. Such calibration can include the use of disposable physical materials that are ablated, with geometric correction and ablation calibration based upon physical impressions left on the physical materials, which are subjectively analyzed by the user. Optical components are replaced when the uniformity of the laser energy across the field becomes unacceptable. At present, typically a 10% variation triggers replacement, and this level can be considered unacceptable in some systems.

Existing methods try to maintain pulse-to-pulse uniformity, which requires a great deal of calibration and testing, limiting the number of surgeries that can be performed, and demanding frequent changing of optical components. Further, the laser must often be purged of its gas, owing to the amount of testing and the requirement for constant power.

DE10335303 is directed to measurement of the irradiation intensity distribution of a laser beam by measurement of intensity of fluorescence of a fluorescent glass which is disposed at a predetermined position for irradiation by the laser beam. In a preferred arrangement the glass is formed to resemble the shape of a cornea. Calibration is performed in a separate mode to operation. Similarly, US2005/021011 relates to a system for the measurement of beam energy, including a monitor located off the optical path of the laser beam to the cornea. A small proportion of the laser beam is transmitted to the monitor while a greater proportion is reflected to the target at the corneal plane.

Therefore, it would be desirable to provide a system for assessing the performance of a refractive laser system that is accurate and automated and eliminates or reduces the above problems associated with prior art refractive laser systems.

### SUMMARY OF THE INVENTION

The invention provides a system in accordance with claim 1.

One embodiment of the system for assessing a performance of a laser system for use in corneal ablation according to the present invention comprises a fluorescent indicator that is adapted to emit a first wavelength of light different from a second wavelength of light impinging thereon. An optical system operates to direct a beam of laser shots onto the indicator in a plane of a cornea of an eye desired to be ablated and also onto a cornea positioned at the corneal plane.

A camera is positioned to detect light reflected from the indicator. An analyzer in signal communication with the camera is provided for calculating a difference between a detected light pattern from the camera and a predetermined ablation pattern desired to be made on the cornea. The analyzer is also provided for correcting the predetermined pattern to compensate for the calculated difference.

Advantageous feature are provided in claims 2-8.

The features that characterize the embodiments of this invention, both as to organization and method of operation, together with further objects and advantages thereof, will be better understood from the following description used in conjunction with the accompanying drawing. It is to be expressly understood that the drawing is for the purpose of illustration and description and is not intended as a definition of the limits of the invention. These and other objects attained, and advantages offered, by the present invention will become more fully apparent as the description that now follows is read in conjunction with the accompanying drawing.

### BRIEF DESCRIPTION OF THE DRAWING

FIG. 1 is a schematic of an embodiment of the system of the present invention;
FIG. 2 is a side perspective view of a monitoring fixture according to the present invention;
FIG. 3 is a flow diagram of an exemplary method of use of the present invention;
FIG. 4 is a camera image of an exemplary grid of laser pulses;
FIG. 5 is an exemplary grid of an input laser pattern versus observed centroids;
FIG. 6 illustrates a user being able to select a particular shot for analysis according to one embodiment of the present invention;
FIG. 7 illustrates a three-dimensional display of the selected shot from FIG. 7, along with a calculation of the shot volume;
FIG. 8 is a three-dimensional view of an average of 20 laser pulses;
FIG. 9 is a graph of the movement of individual centroids from the mean;
FIG. 10 is a graph of normalized volume for a system with a known lower-energy location; and
FIGS. 11A- 11E indicate steps in a method for centering laser shots : FIG. 11A indicates the input pattern; FIG. 11B, image collection; FIG. 11C, the summation of the laser spots; FIG. 11D, plotting of the centroids; and FIG. 11E, translation and rotation of centroids.

### DETAILED DESCRIPTION OF THE INVENTION

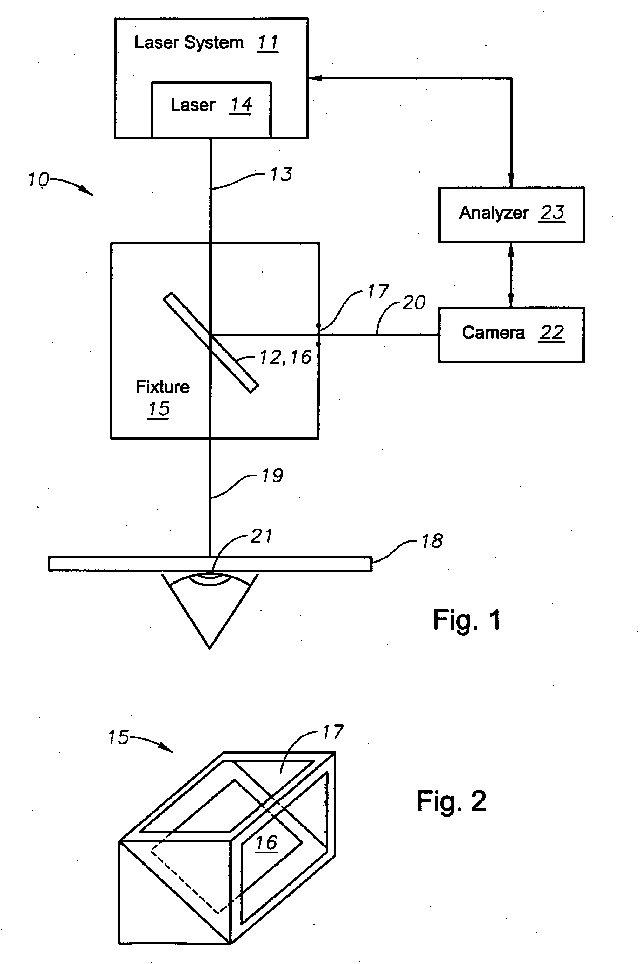

A description of various embodiments of the present invention will now be presented with reference to FIGS. 1-11E.

A system 10 (FIG. 1) for assessing a performance of a laser system 11 is provided for use in corneal ablation, for example. The system 10 comprises a fluorescent indicator 12 that is adapted to emit a first wavelength of light different from a second wavelength of light impinging thereon. A beam 13 from a laser 14 in the laser system 11 is directed via optics to an exemplary embodiment of a fixture 15 for supporting the indicator 12. One embodiment of fixture 15 which comprises a 45-degree, partially reflecting surface 16 and a fluorescent window 17 is shown in more detail in FIG. 2. The partial mirror 16 can comprise ultraviolet (uv) glass, which allows ablation to take place at the eye plane 18 with the transmitted beam 19 while monitoring reflected light 20. The optical system thus operates to direct a beam 13 of laser shots onto the indicator 12/16 in a plane of a cornea of an eye desired to be ablated and also onto a cornea 21 positioned at the corneal plane, both the through-beam 19 and the reflected beam 20 impinging on their respective planes with substantially normal incidence.

A camera 22 is positioned to detect the light 20 reflected from the indicator. An analyzer 23 in signal communication with the camera 22 is provided for calculating a difference between a detected light pattern from the camera 22 and a predetermined ablation pattern desired to be made on the cornea 21. The analyzer 23 is also provided for correcting the predetermined pattern to compensate for the calculated difference. Preferably the camera 22 and the analyzer 23 are adapted to operate faster than a shot rate of the laser 14.

In a particular embodiment, the camera 22 has asynchronous and synchronous modes. The asynchronous mode synchronizes to an external input for the start of frame. The integration time is set by an internal register/timing. The maximum frame rate is approximately 50 Hz. If the synch signal exceeds the maximum frame rate, the camera waits for the next synch. An existing laser pretrigger pulse for the system gives adequate time for camera reset. As shown by Table 1, the duty cycle can be determined by the laser pulse rate and the pulse capture rate.

| Table 1. Duty Cycle as a Function of Laser Pulse Rate and Pulse Capture Rate | | |
|---|---|---|
| Laser Pulse Rate (Hz) | Pulse Capture Rate (Hz) | Duty Cycle |
| 30 | 30 | 30 |
| 45 | 45 | 1 |
| 60 | 30 | ½ |
| 80 | 40 | ½ |
| 105 | 35 | 1/3 |

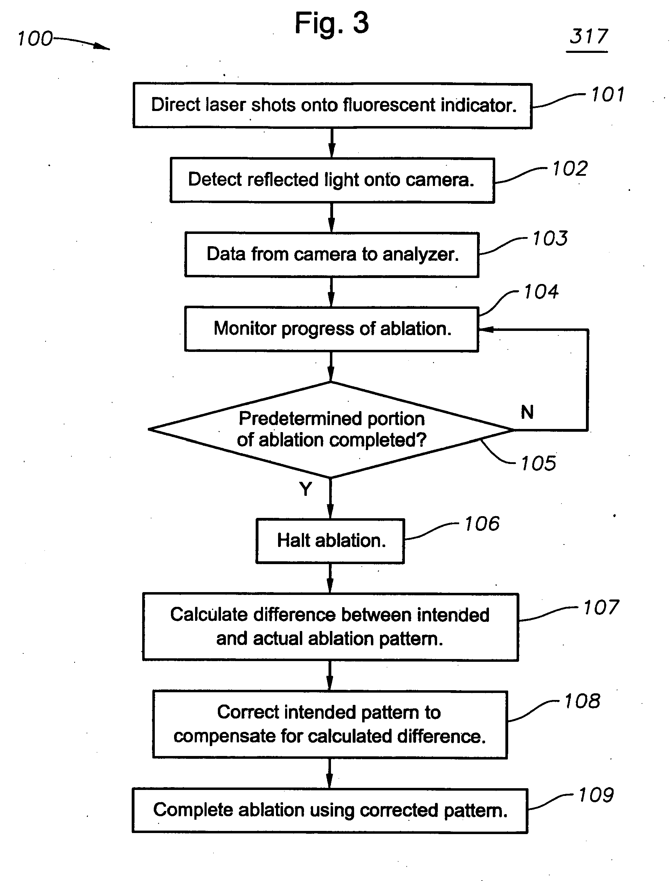

A method 100 for assessing a performance of a laser system for use in corneal ablation includes directing a beam of laser shots onto a fluorescent indicator 12 as above (block 101; FIG. 3). Light reflected from the indicator 12 is detected by the camera 22 (block 102) at a rate faster than a shot rate of the laser 14.

The camera 22 sends data to an analyzer 23 (block 103), where a difference between a detected light pattern from the camera and a predetermined ablation pattern desired to be made on the cornea is calculated. The analyzer 23 can then correct the predetermined pattern to compensate for the calculated difference.

In a particular embodiment of a method for carrying out a corneal ablation, the progress of the creation of the predetermined pattern is monitored (block 104). When a predetermined portion of the predetermined pattern has been completed (block 105), the ablation is halted (block 106), and the difference between the portion of the predetermined pattern that has been completed and the detected light pattern is calculated (block 107). Then the remaining portion of the predetermined pattern is corrected to make up for the difference (block 108), and the ablation pattern is completed (block 109).

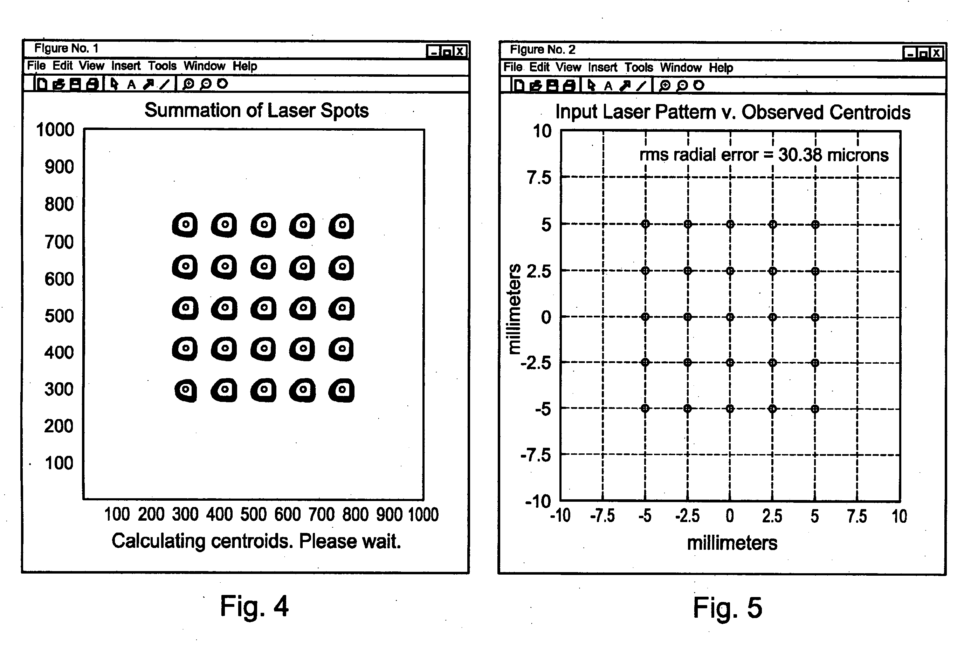

In FIG. 4 is depicted an exemplary camera image of a grid of laser pulses, and in FIG. 5, a display of the input laser pattern versus the observed centroids calculated from the data of FIG. 4. The shots, which are intended to be placed at the intersection of the grid lines, can be seen to deviate slightly from their desired positions, and an rms radial error of 30.38 µm is found.

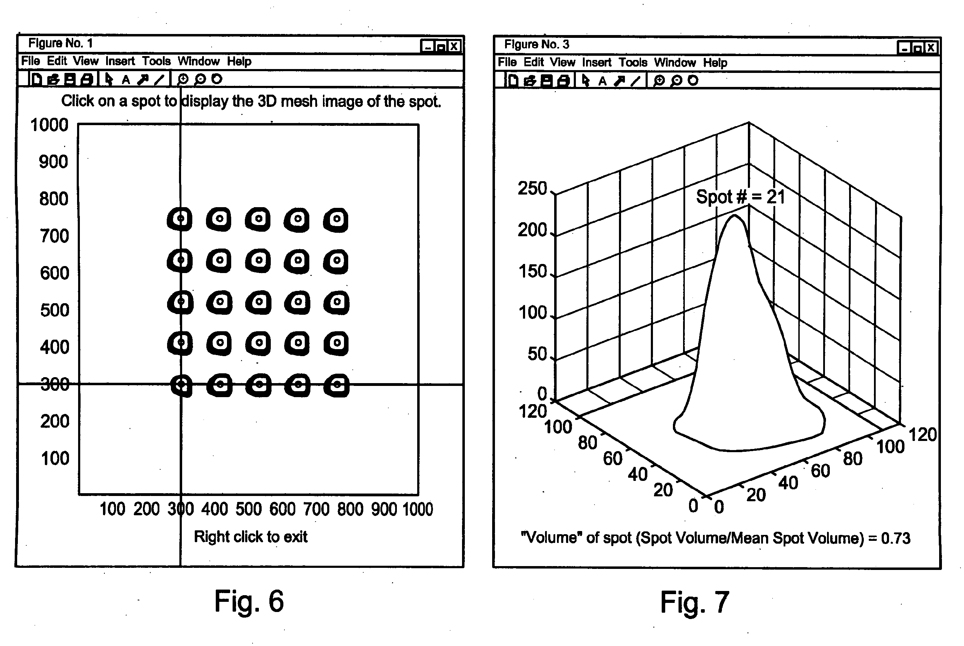

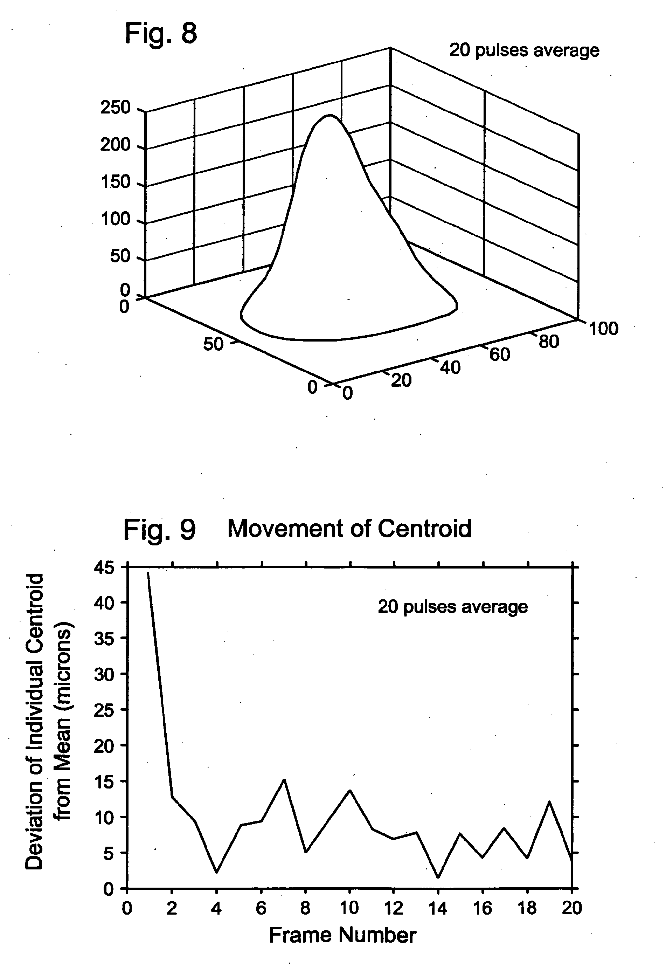

FIG. 6 illustrates a user being able to select a particular shot for analysis on a display screen of a matrix of shots. FIG. 7 illustrates a three-dimensional display of the selected shot from FIG. 6, along with a calculation of the shot volume. FIG. 8 is a three-dimensional view of an average of 20 laser pulses, and FIG. 9 is a graph of the movement of individual centroids from the mean.

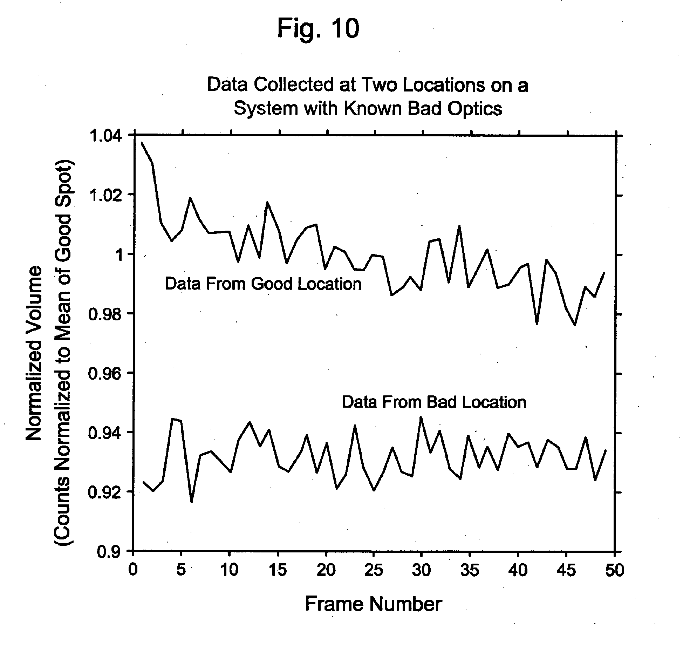

The performance of a laser system can be estimated using the system 10 of the present invention. As an experiment, a system with known bad optics at a particular location was measured to have a variation of 7% using an energy meter, which correlates well with a measurement using the present system of 93% of the energy at the less efficient location. The experiment was performed by collecting 50 sequential frames of data at a "good" spot and a "bad" spot (FIG. 10). The volume is found by summing the pixel values, and the volumes are normalized to the mean volume at the good spot. The mean value at the bad spot is 93% of the mean value at the good spot, with a standard deviation of the mean at the good spot of ~1.2% and a standard deviation of the mean at the bad spot of ~0.7%.

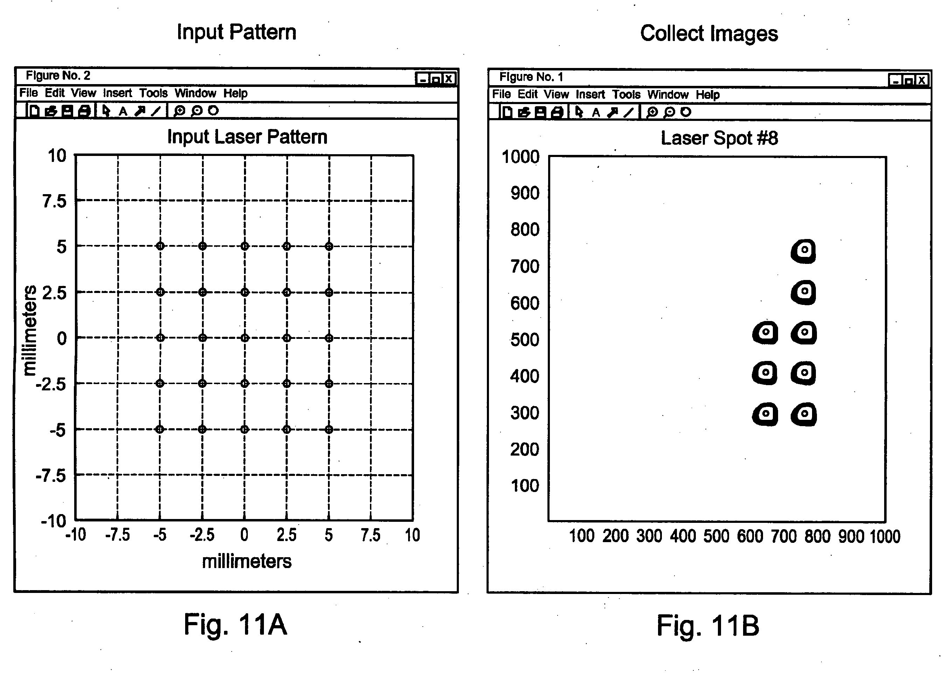

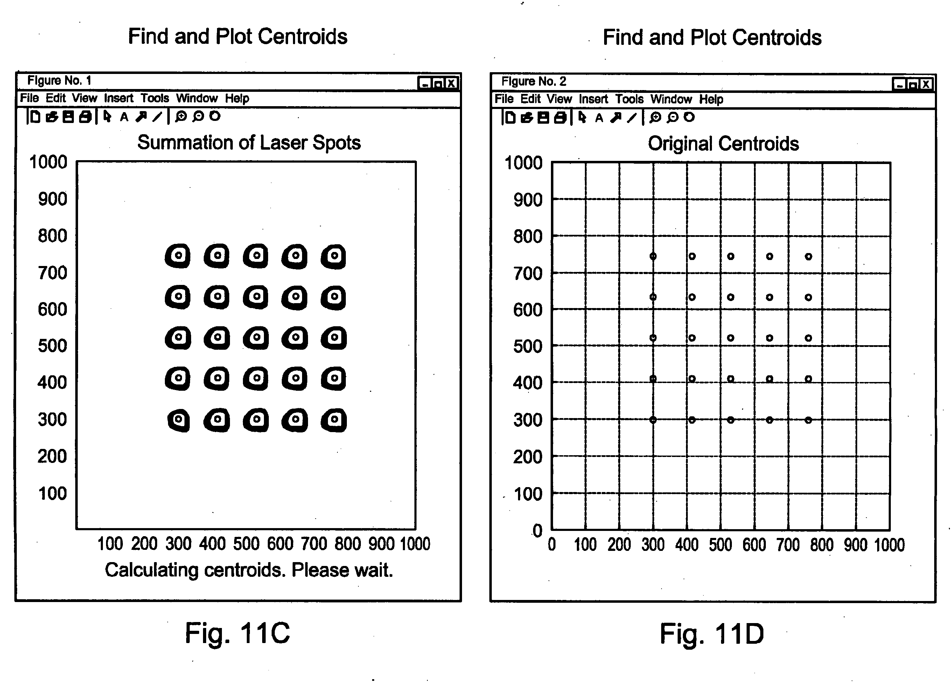

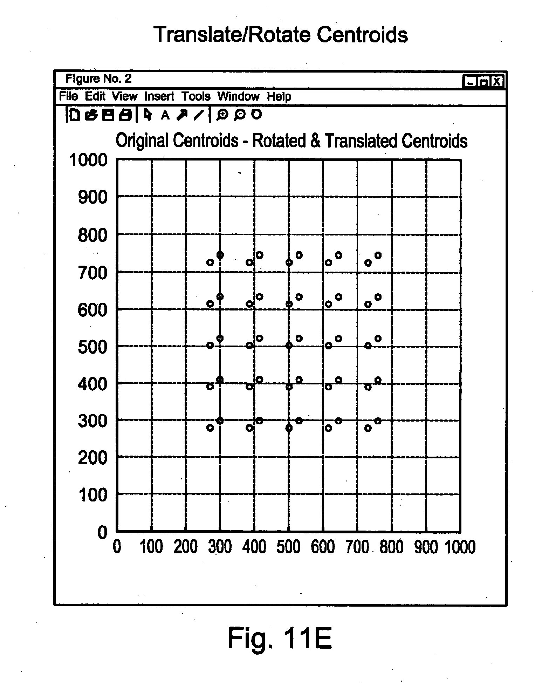

FIGS. 11A-11E indicate steps in a method for centering laser shots. FIG. 11A indicates the input pattern; FIG. 11B, image collection; FIG. 11C, the summation of the laser spots; FIG. 11D, plotting of the centroids; and FIG. 11E, translation and rotation of centroids.

The system 10 described in the exemplary embodiments herein can be seen to provide multiple points for detecting more than just rotation errors as known previously. Unlike the typical paper ablation known in the art, a long-lived fluorescent material is used. The fluorescent uniformity is not critical. Pulse averaging can be performed for laser power variation, but is not necessary for geometric calculations. Another important feature is that individual laser spots can be viewed to detect laser motion at a single location, and the average laser shape can be analyzed to estimate shot shape and power.

In the foregoing description, certain terms have been used for brevity, clarity, and understanding, but no unnecessary limitations are to be implied therefrom beyond the requirements of the prior art, because such words are used for description purposes herein and are intended to be broadly construed. Moreover, the embodiments of the apparatus illustrated and described herein are by way of example, and the scope of the invention is not limited to the exact details of construction.

Having now described the invention, the construction, the operation and use of preferred embodiments thereof, and the advantageous new and useful results obtained thereby, the new and useful constructions, and reasonable mechanical equivalents thereof obvious to those skilled in the art, are set forth in the appended claims.

## Claims

1. A system (10) for assessing a performance of a laser system (11) for use in corneal ablation, the system comprising:
a fluorescent indicator (12) adapted to emit a first wavelength of light different from a second wavelength of light impinging thereon;
a camera (22) positioned to detect light reflected (20) from the indicator;
an optical system (15) for directing a beam (13) of laser (14) shots onto a cornea positioned at the corneal plane (18) the laser beam(13) being directed substantially normally onto the cornea, and also directed onto the indicator, **characterized by** the indicator being substantially located between the laser and cornea on the optical path of the laser beam (13) to the cornea, and by comprising
an analyzer (23) in signal communication with the camera for calculating a difference between a detected light pattern from the camera and a predetermined ablation pattern desired to be made on the cornea, based on calculating centroids of observed pulses of the detected light pattern and comparing their positions with a grid representation of the predetermined pattern, and for correcting the predetermined pattern to compensate for the calculated difference.

2. The system recited in Claim 1, wherein the optical system comprises a partial mirror (16) adapted to reflect the first and the second wavelength of light, the partial mirror oriented at approximately 45 degrees to the laser beam (13), and the cornea (21) is positioned substantially normal to the laser beam.

3. The system recited in Claim 2, wherein the partial mirror (16) comprises an ultraviolet glass material.

4. The system recited in Claim 2, wherein the partial mirror (16) is coated with a material adapted to pass the laser beam (13) and reflect visible fluorescence.

5. The system recited in Claim 1, wherein the camera (22) and the analyzer (23) are adapted to operate faster than a shot rate of the laser (14).

6. The system recited in Claim 5, wherein the analyzer (23) is adapted to calculate an energy profile of a single laser shot.

7. The system recited in Claim 6, wherein the analyzer (23) is adapted to calculate an ablated volume of a unitary laser shot.

8. The system recited in Claim 1, further comprising a controller in signal communication with the optical system for halting the beam (13) of laser (14) shots when a predetermined portion of the predetermined pattern has been completed, and wherein the analyzer is adapted to calculate a difference between the portion of the predetermined pattern that has been completed and the detected light pattern, and to perform the correcting compensation on a remaining portion of the predetermined pattern.

## Patentansprüche

1. System (10) zur Bewertung der Leistung eines Lasersystems (11), das bei der Kornea-Ablation verwendet wird, wobei das System aufweist:
eine fluoreszierende Anzeigeeinrichtung (12), die dazu eingerichtet ist, Licht einer ersten Wellenlänge, das verschieden ist von Licht einer zweiten Wellenlänge, das auf diese fällt, zu emittieren;
eine Kamera (22), die so positioniert ist, dass sie das von der Anzeigeeinrichtung reflektierte Licht (20) erfasst;
ein optisches System (15) zum Richten eines Strahls (13) aus Pulsen des Lasers (14) auf die Kornea, die in der Kornea-Ebene (18) des im Wesentlichen senkrecht auf die Kornea gerichteten Laserstrahls (13) positioniert ist, und der auch auf die Anzeigeeinrichtung gerichtet ist,
**dadurch gekennzeichnet, dass** die Anzeigeeinrichtung im Wesentlichen zwischen dem Laser und der Kornea auf dem optischen Weg des Laserstrahls (13) zur Kornea angeordnet ist, und dass
es einen Analysator (23) aufweist, der mit der Kamera Signale austauscht, um die Differenz zwischen einem erfassten Lichtmuster von der Kamera und einem vorgegebenen Ablations-Muster auf Basis der Berechnung von Schwerpunkten der beobachteten Pulse des erfassten Lichtmusters zu berechnen, das auf der Kornea ausgeführt werden soll, und zum Vergleichen ihrer Positionen mit einer Gitterdarstellung des vorgegebenen Musters und zur Korrektur des vorgegebenen Musters, um die berechnete Differenz zu kompensieren.

2. System nach Anspruch 1, bei dem das optische System einen partiellen Spiegel (16) aufweist, der dazu einrichtet ist, das Licht mit der ersten und zweiten Wellenlänge zu reflektieren, wobei der partielle Spiegel im Winkel von ca. 45° zum Laserstrahl (13) ausgerichtet und die Kornea (21) im Wesentlichen senkrecht zum Laserstrahl positioniert ist.

3. System nach Anspruch 2, bei dem der partielle Spiegel (16) ein ultraviolettes Glasmaterial aufweist.

4. System nach Anspruch 2, bei dem der partielle Spiegel (16) mit einem Material beschichtet ist, das zum Durchlassen des Laserstrahls (13) und zum Reflektieren der sichtbaren Fluoreszenz eingerichtet ist.

5. System nach Anspruch 1, bei dem die Kamera (22) und der Analysator (23) für einen schnelleren Betrieb als die Pulsrate des Lasers (14) eingerichtet sind.

6. System nach Anspruch 5, bei dem der Analysator (23) zum Berechnen eines Energieprofils eines einzelnen Laserpulses eingerichtet ist.

7. System nach Anspruch 6, bei dem der Analysator (23) zum Berechnen eines Ablationsvolumens eines unitären Laserpulses eingerichtet ist.

8. System nach Anspruch 1, das ferner eine Steuerung aufweist, die mit dem optischen System Signale austauscht, um den Strahl (13) aus Pulsen des Lasers (14) zu unterbrechen, wenn ein vorgegebener Abschnitt des vorgegebenen Musters ausgeführt worden ist, und bei dem der Analysator dazu eingerichtet ist, die Differenz zwischen dem Abschnitt des vorgegebenen Musters, der ausgeführt worden ist, und dem erfassten Lichtmuster zu berechnen und am verbleibenden Abschnitt des vorgegebenen Musters die korrektive Kompensation auszuführen.

## Revendications

1. Système (10) pour évaluer la performance d'un système laser (11) destiné à être utilisé pour une ablation de la cornée, le système comprenant :
un indicateur fluorescent (12) adapté pour émettre une première longueur d'onde de lumière différente d'une seconde longueur d'onde de lumière empiétant sur celle-ci ;
une caméra (22) positionnée pour détecter une lumière réfléchie (20) depuis indicateur,
un système optique (15) pour diriger un faisceau (13) de décharges laser (14) sur une cornée positionnée au niveau du plan cornéen (18), le faisceau laser (13) étant dirigé sensiblement perpendiculairement sur la cornée, et dirigé également sur indicateur, **caractérisé en ce que** l'indicateur est sensiblement situé entre le laser et la cornée sur le trajet optique du faisceau laser (13) vers la cornée, et **en ce qu'**il comprend :
un analyseur (23) en communication de signaux avec la caméra pour calculer une différence entre un motif de lumière détecté provenant de la caméra et un motif d'ablation prédéterminé censé être réalisé sur la cornée, sur la base du calcul de centroïdes des impulsions observées du motif de lumière détecté, et sur la base de la comparaison de leurs positions à une représentation en grille du motif prédéterminé, et pour corriger le motif prédéterminé afin de compenser la différence calculée.

2. Système selon la revendication 1, dans lequel le système optique comprend un miroir partiel (16) adapté pour réfléchir la première et la seconde longueurs d'onde de lumière, le miroir partiel étant orienté à environ 45 degrés par rapport au faisceau laser (13), et la cornée (21) étant positionnée sensiblement perpendiculairement au faisceau laser.

3. Système selon la revendication 2, dans lequel le miroir partiel (16) comprend un matériau en verre ultraviolet.

4. Système selon la revendication 2, dans lequel le miroir partiel (16) est recouvert d'un matériau adapté pour laisser passer le faisceau laser, (13), et pour réfléchir une fluorescence visible.

5. Système selon la revendication 1, dans lequel la caméra (22) et l'analyseur (23) sont adaptés pour fonctionner plus vite que la vitesse de décharge du laser (14).

6. Système selon la revendication 1, dans lequel l'analyseur (23) est adapté pour calculer un profil énergétique d'une décharge laser unique.

7. Système selon la revendication 6, dans lequel l'analyseur (23) est adapté pour calculer le volume enlevé d'une décharge laser unitaire.

8. Système selon la revendication 1, comprenant en outre un dispositif de commande en communication de signaux avec le système optique pour interrompre le faisceau (13) de décharges laser (14) lorsqu'une partie prédéterminée du motif prédéterminé a été achevée, et dans lequel l'analyseur est adapté pour calculer une différence entre la partie du motif prédéterminée qui a été achevée et le motif de lumière détecté, et pour effectuer la compensation correctrice sur une partie restante du motif prédéterminé.
